# EUROPÄISCHE PATENTSCHRIFT

(11) **EP 2 221 083 B1**
(45) Veröffentlichungstag und Bekanntmachung des Hinweises auf die Patenterteilung: **09.05.2012**
(21) Anmeldenummer: 10151271.3
(22) Anmeldetag: 21.01.2010
(51) Int. Cl.: A61M 27/00

(54) **Ventil zum Einsatz in einer Leitung zum Führen einer Flüssigkeit**
Valve for use in a conduit for guiding a liquid
Soupape à utiliser dans une conduite pour diriger un liquide

(30) Priorität: 20.02.2009 DE 102009009880
(43) Veröffentlichungstag der Anmeldung: 25.08.2010
(73) Patentinhaber: RAUMEDIC AG, 95213 Münchberg (DE)
(72) Erfinder: Kunze, Hans Gerd, 08297, Zwönitz (DE); von Falkenhausen, Christian, 53340, Meckenheim (DE); Ansorge, Frank, 81245, München (DE); Irlbauer, Markus, 82319, Starnberg (DE)
(74) Vertreter: Hofmann, Stefan

(56) Entgegenhaltungen:
- EP-A2- 0 414 649
- EP-A2- 0 873 762
- FR-A1- 2 896 422
- US-A1- 2006 052 737
- US-B1- 6 432 050

## Beschreibung

Die Erfindung betrifft ein Ventil, insbesondere ein medizinisches Ventil, zum Einsatz in einer Leitung zum Führen einer Flüssigkeit aus einer Kammer, insbesondere aus einer Körperhöhlung, insbesondere zum Einsatz in einer Leitung zum Abführen von Liquor aus einer Hirnkammer.

Ein derartiges Ventil ist beispielsweise aus der CH 684 708 A5 bekannt. Ein weiteres Ventil ist aus der EP 0 334 157 A2 bekannt. Aus der FR 2 896 422 A1 ist ein Hydrozephalus-Drainageventil bekannt. Aus der US 2006/0052737A1 sind verschiedene Varianten eines Drainagesystems bekannt, zu dem neben einem ventrikulären Katheter und einem Drainagekatheter eine Pumpe gehört, die aktiv CSF (cerebral spinal fluid) aus dem Himbereich eines Patienten abführt.

Es ist eine Aufgabe der vorliegenden Erfindung, ein Ventil der eingangs genannten Art derart weiterzubilden, dass eine unnötige Ableitung von Flüssigkeit, insbesondere eine unnötige Drainage von Liquor, vermieden ist.

Diese Aufgabe ist erfindungsgemäß gelöst durch ein Ventil mit den im Anspruch 1 angegebenen Merkmalen.

Erfindungsgemäß wurde erkannt, dass ein Überschreiten der Flüssigkeitsdruck-Obergrenze zumindest dann, wenn die Obergrenze nicht über einen längeren Zeitraum überschritten bleibt, kein Öffnen des Ventils und damit beispielsweise keine Flüssigkeitsdrainage erfordert. Beim Einsatz in einer Leitung zum Abführen von Liquor aus einer Hirnkammer führen normale Liquordruck-Anstiege, beispielsweise durch ein Valsalva-artiges Manöver oder beim Husten, dann nicht zu einer unnötigen Liquordrainage, da das Ventil geschlossen bleibt. Ein weitgehendes Verschließen des Ventils bedeutet, dass bei geschlossenem Ventil nicht zwingend überhaupt kein Flüssigkeits-Fluss zwischen dem Ventilsitz und dem Ventilkörper mehr möglich ist. Ein im Vergleich zum geöffneten Ventil geringer Flüssigkeitsstrom, beispielsweise 10% oder weniger als beim geöffneten Ventil, kann beim weitgehenden Verschließen des Ventils noch möglich sein. Das Ventil gewährleistet, dass dann, wenn die Flüssigkeitsdruck-Obergrenze während eines vorgegebenen Zeitraums überschritten bleibt, eine Drainage stattfindet. Hierdurch kann ein normaler, kurzzeitiger und keine Drainage erfordernder Flüssigkeitsdruck-Anstieg von einem pathologischen und eine Drainage erfordernden Flüssigkeitsdruck-Anstieg unterschieden werden.

Das erfindungsgemäße Ventil hat einen Niederdruck-Ventilsitz, der mit einem Ventilkörper derart zusammenwirkt, dass das Ventil unterhalb der Flüssigkeitsdruck-Untergrenze geschlossen ist, und einen Hochdruck-Ventilsitz, der mit einem Ventilkörper derart zusammenwirkt, dass das Ventil oberhalb der Flüssigkeitsdruck-Obergrenze zumindest zeitweilig und zumindest weitgehend geschlossen ist. Eine derartige Ausgestaltung des Ventils mit einem Niederdruck-Ventilsitz und einem Hochdruck-Ventilsitz ist konstruktiv einfach möglich.

Eine Ausgestaltung nach Anspruch 2 ist konstruktiv besonders einfach. Beim Ventilkörper handelt es sich insbesondere um eine Kugel.

Eine Vorspannfeder nach Anspruch 3 führt zu einer sauberen Vorgabe der Flüssigkeitsdruck-Untergrenze. Entsprechend lässt sich über die Vorspannfeder auch die Flüssigkeitsdruck-Obergrenze vorgeben. Dies kann über die Auslegung der Federkraft sowie über die Geometrie des Ventilkörpers und die Anordnung der Ventilsitze zueinander, beispielsweise den Abstand der Ventilsitze zueinander, geschehen.

Eine expandierbare Kammer nach Anspruch 4 wirkt als Zeitglied. Die bei Überschreiten der Flüssigkeitsdruck-Obergrenze bei dieser Gestaltung zunächst geschlossene Kammer-Ventileinheit mit dem Kammer-Ventilkörper und dem Kammer-Ventilsitz wirkt als durch den die Kammer füllenden Flüssigkeit verlagerbarer Kolben, wobei der Kolben nur während einer vorgegebenen Zeitspanne des Überschreitens der Flüssigkeitsdruck-Obergrenze dicht bleibt. Ein solcher Kolben kann auch als Dämpfungsglied bezeichnet werden.

Eine Vorspanneinrichtung nach Anspruch 5 stellt ein Rückstellen der expandierbaren Kammer in Richtung der nicht expandierten Kammerstellung sicher, sobald der Flüssigkeitsdruck die vorgegebene Obergrenze wieder unterschreitet. Bei den Kammer-Ventilelementen handelt es sich um den Kammer-Ventilkörper einerseits und den Kammer-Ventilsitz andererseits. Es können beide Kammer-Ventilelemente über die gleiche Vorspanneinrichtung oder auch über diesen jeweils zugeordnete individuelle Vorspanneinrichtungen in Richtung auf die nicht expandierte Stellung der Kammer zu vorgespannt sein.

Mechanische Verbindungen nach den Ansprüchen 6 und 7 vereinfachen die konstruktive Gestaltung des Ventils.

Eine Relativbewegung nach Anspruch 8 erleichtert eine konstruktive Auslegung der Kammer-Ventileinheit mit dem Kammer-Ventilsitz und dem Kammer-Ventilkörper, bei der diese während einer Kammerexpansion zunächst geschlossen bleibt. Bei der Vorspanneinrichtung, die das mit dem Hochdruck-Ventilelement mechanisch verbundene Kammer-Ventilelement in Richtung auf einen dem Hochdruck-Ventilelement zugewandten Anschlag vorspannt, kann es sich um die Vorspanneinrichtung handeln, die zumindest eines der Kammer-Ventilelemente in Richtung auf die nicht expandierte Stellung der Kammer zu vorspannt. Bei dem Hochdruck-Ventilelement handelt es sich um den Hochdruck-Ventilsitz oder um den Hochdruck-Ventilkörper.

Eine Verriegelungsvorrichtung nach Anspruch 9 gewährleistet definierte Betriebsstellungen des Ventils. Die Verlagerung des Hochdruck-Ventilsitzes relativ zum Hochdruck-Ventilkörper kann längs der Flüssigkeits-Strömungsrichtung erfolgen.

Eine Entriegelungseinrichtung nach Anspruch 10 gewährleistet eine kontrollierte Überführung der Verriegelungsvorrichtung von der Sperrstellung in die Freigabestellung zum Öffnen des Ventils, sobald die Flüssigkeitsdruck-Obergrenze länger als der vorgegebene Zeitraum überschritten ist. Die Entriegelungseinrichtung kann insbesondere aufeinander zur Verlagerung eines Riegels gleitende Keilabschnitte aufweisen.

Eine Vorspannfeder nach Anspruch 11 gewährleistet ein kontrolliertes Entriegeln der Verriegelungsvorrichtung.

Eine Blattfeder nach Anspruch 12 ermöglicht eine konstruktiv einfache Auslegung der Verriegelungsvorrichtung.

Eine Einstellbarkeit nach Anspruch 13 gewährleistet eine Anpassung der vorgegebenen Werte für die Flüssigkeitsdruck-Untergrenze und die Flüssigkeitsdruck-Obergrenze an die jeweiligen Patientenerfordernisse. Obwohl das Ventil nachfolgend im Zusammenhang mit einem speziellen medizinischen Einsatzzweck erläutert wird, ist der Einsatz des erfindungsgemäßen Ventils nicht auf eine medizinische Anwendung beschränkt. Auch in anderen, nicht medizinischen Anwendungsfeldern kann das Ventil zum Einsatz kommen, soweit eine unnötige Flüssigkeitsabführting vermieden werden soll.

Ein Ausfühnmgsbeispiel der Erfindung wird nachfolgend anhand der Zeichnung näher erläutert. In dieser zeigen:
- Fig. 1: schematisch die Anordnung eines medizinischen Ventils zum Einsatz in einer Leitung zum Abführen von Liquor aus einer Hirnkammer in einen Bauchraum eines Patienten;
- Fig. 2: schematisch in einem axialen Längsschnitt das medizinische Ventil in einem Betriebszustand unterhalb einer vorgegebe- nen Liquordnick-Untergrenze;
- Fig. 3: in einer zu Figur 2 ähnlichen Darstellung das Ventil in einem Betriebszustand kurz nach dem Überschreiten einer Li- quordnick-Obergrenze;
- Fig. 4: in einer zu Figur 2 ähnlichen Darstellung das Ventil in einem Betriebszustand nach längerem Überschreiten der vorgegebe- nen Liquordruck-Obergrenze; und
- Fig. 5: in einer zu Figur 2 ähnlichen Darstellung das Ventil in einem Betriebszustand, bei dem nach längerem Überschreiten der Liquordruck-Obergrenze diese wieder unterschritten ist und das Ventil zum Ausgangszustand nach Figur 2 zurückkehrt.

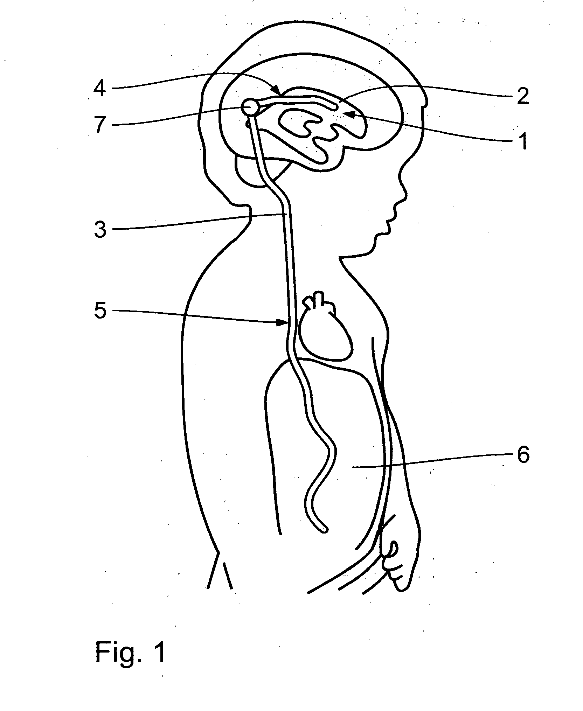

Figur 1 zeigt schematisch einen Hydrozephalus-Patienten. Ein Ventrikelsystem 1 mit einem Liquorraum (Hirnkammer) 2 als Beispiel für eine Flüssigkeitskammer, nämlich für eine mit Flüssigkeit gefüllte Körperhöhlung, steht über eine Liquorleitung 3 mit einem proximalen Leitungsabschnitt 4 und einem distalen Leitungsabschnitt 5 mit einem Bauchraum 6 in Fluidverbindung. In der Liquorleitung 3 ist ein medizinisches Ventil 7 angeordnet. Letzteres dient zum gezielten Abführen von Liquor als Beispiel für eine Flüssigkeit aus dem Liquorraum (Hirnkammer) 2.

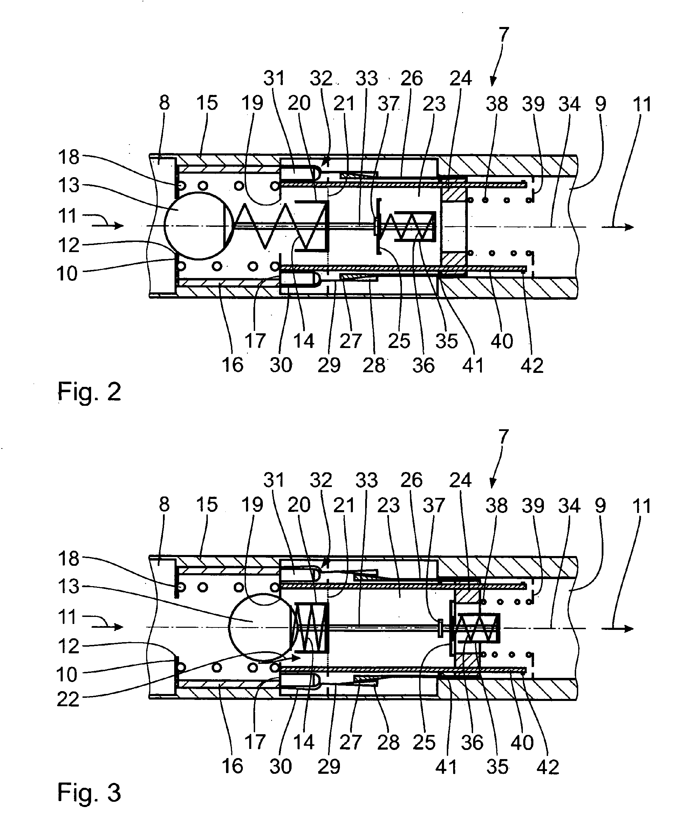

Figuren 2 bis 5 zeigen Details des Ventils 7 in verschiedenen, über den Liquordruck jeweils ausgelösten Betriebszuständen. Ein stromaufseitiger Abschnitt 8 des Ventils 7 steht mit dem proximalen Leitungsabschnitt 4 und ein stromabseitiger Abschnitt 9 des Ventils 7 steht mit dem distalen Leitungsabschnitt 5 in Fluidverbindung.

Das Ventil 7 ist so ausgeführt, dass es zwischen einer vorgegebenen Liquordnick-Untergrenze und einer vorgegebenen Liquordruck-Obergrenze im stromaufseitigen Abschnitt 8 geöffnet und beim Unterschreiten der Liquordruck-Untergrenze einerseits und beim Überschreiten der Liquordruck-Obergrenze andererseits geschlossen ist.

Das Ventil 7 hat einen stromaufseitigen Niederdruck-Ventilsitz 10, der als senkrecht zu einer Liquor-Ströhmungsrichtung 11 verlaufende Wand mit einer zentralen Durchgangsöffnung 12 ausgebildet ist. Der Niederdruck-Ventilsitz 10 wirkt mit einem Ventilkörper 13 derart zusammen, dass das Ventil 7 unterhalb der Liquordruck-Untergrenze geschlossen ist. Der Ventilkörper 13 ist als Kugel ausgebildet, deren Durchmesser größer ist als der Durchmesser der runden Durchgangsöffnung 12. Der Ventilkörper 13 ist über eine Ventilkörper-Vorspannfeder 14 entgegen der Liquor-Strömungsrichtung 11 in Richtung auf den Niederdruck-Ventilsitz 10 vorgespannt. Beim Überschreiten der Liquordruck-Untergrenze im stromaufseitigen Abschnitt 8 übersteigt der Liquordruck, der den Ventilkörper 13 in der Figur 2 von links beaufschlagt, den Gegendruck, den die Ventilkörper-Vorspannfeder 14 auf den Ventilkörper 13 ausübt, so dass der Ventilkörper 13 vom Niederdruck-Ventilsitz 10 zum Öffnen des Ventils 7 abhebt.

Der Niederdnick-Ventilsitz 10 stützt sich an einer Umfangsstufe einer äußeren Gehäusewand 15 des Ventils 7 ab. In die äußere Gehäusewand 15 eingepasst ist eine Sitzhülse 16, die an ihrem stromabseitigen Ende einen Hochdruck-Ventilsitz 17 trägt. Zwischen den beiden Ventilsitzen 10, 17 stützt sich eine Druckfeder 18 ab, die in noch zu beschreibender Weise als Hochdruck-Ventilsitz-Vorspannfeder dient.

Der Hochdruck-Ventilsitz 17 weist fluchtend mit derNiederdruck-Durchgangsöffnung 12 eine Hochdruck-Durchgangsöffnung 19 auf.

Der Hochdruck-Ventilsitz 17 wirkt mit dem Ventilkörper 13 derart zusammen, dass das Ventil 7 oberhalb der Liquordruck-Obergrenze zumindest zeitweilig weitgehend geschlossen ist, so dass bei auf dem Hochdruck-Ventilsitz 17 aufliegendem Ventilkörper 13 maximal noch 1/10 des Liquorflusses vorliegt, der bei geöffnetem Ventil 7 möglich ist.

Der Ventilkörper 13 wirkt je nach Liquordruck mit dem Niederdruck-Ventilsitz 10 oder mit dem Hochdruck-Ventilsitz 17 zusammen. Soweit der Liquordruck zwischen der Liquordruck-Untergrenze und der Liquordruck-Obergrenze liegt, liegt der Ventilkörper 13 zwischen den beiden Ventilsitzen 10, 17 und das Ventil 7 ist geöffnet.

Die Ventilkörper-Vorspannfeder 14 stützt sich mit ihrem vom Ventilkörper 13 abgewandten Ende an einem Stütztopf 20 ab, der über ein Durchlassgitter 21 axial an der äußeren Gehäusewand 15 festgelegt ist.

Beim Überschreiten der Liquordruck-Obergrenze bleibt das Ventil 7 eine vorgegebene Zeitspanne geschlossen. Bei anhaltendem Überschreiten der Liquordruck-Obergrenze öffnet das Ventil 7 wieder.

Figur 3 zeigt die Betriebsstellung des Ventils 7 nach kurzzeitigem Überschreiten der Liquordruck-Obergrenze. Der Ventilkörper 13 liegt am Hochdruck-Ventilsitz 17 an, so dass das Ventil 7 weitgehend geschlossen ist. Wie in der Figur 3 durch einen Strömungspfeil 22 verdeutlicht, ist ein geringer Liquor-Massenstrom, also maximal 1/10 des Liquorflusses bei geöffnetem Ventil 7, auch in der Stellung nach Figur 3 zwischen dem Ventilkörper 13 und dem Hochdrtick-Ventilsitz 17 in eine expandierbare Kammer 23 möglich.

Stromabseitig schließt die Kammer 23 über einen als Kolbenring ausgeführten Kammer-Ventilsitz 24 ab, der mit einem als Deckel ausgebildeten Kammer-Ventilkörper 25 zusammenwirkt.

Der Kammer-Ventilsitz 24 ist an einem stromabseitigen Ende einer im Gehäuse des Ventils 7 untergebrachten Kammerhülse 26 festgelegt. Eine Gehäuse-Wandstärke der Kammerhülse 26 vergrößert sich an deren stromaufseitigem Ende in einen Hülsen-Keilabschnitt 27. Letzterer liegt an einem komplementär hierzu ausgeführten Feder-Keilabschnitt 28 an, der den Hülsen-Keilabschnitt 27 außen umgibt. Der Feder-Keilabschnitt 28 stellt ein Ende einer Blattfeder 29 dar. Die Blattfeder 29 ist unterteilt in eine Mehrzahl von einzelnen, um die Längsachse 34 (vgl. Figur 3) herum angeordnete Federzungen. Diese Federzungen stellen eine symmetrische Kraftverteilung radial nach innen, also zur Längsachse 34 hin, bereit. Es können zwei oder mehr derartige Federzungen in Umfangsrichtung um die Längsachse 34 gleich verteilt angeordnet sein.

Das andere Ende der Blattfeder 29 ist an der äußeren Gehäusewand 15 stromaufseitig festgelegt. Zwischen dem Feder-Keilabschnitt 28 und der äußeren Gehäusewand 15 hat die Blattfeder 29 eine Auswölbung 30. Letztere ist komplementär zu freien Enden von rundköpfigen Zapfen 31 ausgebildet. Die stromaufseitigen Enden der Zapfen 31 sind am Hochdruck-Ventilsitz 17 festgelegt. Die Längsachsen der Zapfen 31 verlaufen parallel zur Liquor-Strömungsrichtung 11.

Die Auswölbung 30 in der Blattfeder 29 stellt zusammen mit den Zapfen 31 eine Verriegelungsvorrichtung 32 dar, die in einer Sperrstellung eine Verlagerung des Hochdruck-Ventilsitzes 17 relativ zum Ventilkörper 13 längs der Liquor-Strönumgsrichtung 11 sperrt und in einer Freigabestellung eine Verlagerung des Hochdruck-Ventilsitzes 17 relativ zum Ventilkörper 13 längs der Liquor-Strömungsrichtung 11 zur Freigabe einer Überströmverbindung zwischen dem Ventilkörper 13 und dem Hochdruck-Ventilsitz 17 freigibt.

Der Kammer-Ventilkörper 25 ist auf einen zentralen Verbindungsstab 33 aufgesteckt, der eine zentrale Öffnung des Kammer-Ventilkörpers 25 durchtritt. Der Verbindungsstab 33 erstreckt sich längs einer zentralen Längsachse 34 des Ventils 7, die parallel zur Liquor-Strömungsrichtung 11 verläuft.

Ein stromaufseitiges Ende des Verbindungsstabes 33 durchdringt eine Durchgangsöffnung des Stütztopfes 20 und ist mit dem Ventilkörper 13 verbunden. Der Ventilkörper 13 ist über den Verbindungsstab 33 mechanisch mit dem Kammer-Ventilkörper 25 verbunden. Ein stromabseitiges Ende des Verbindungsstabes 33 trägt einen weiteren Stütztopf 35, der stromabwärts des Ventilkörper-Stütztopfes 20 angeordnet ist. Zwischen dem Stütztopf 35 und dem Kammer-Ventilkörper 25 stützt sich eine Kammer-Ventilkörper-Vorspannfeder 36 ab, die den Kammer-Ventilkörper 25 in Richtung auf einen Anschlagbund 37 vorspannt, der am Verbindungsstab 33 festgelegt ist.

Eine Kammer-Ventilsitz-Vorspannfeder 38 stützt sich stromabwärts des Kammer-Ventilsitzes 24 zwischen diesem und einem weiteren stromabseitigen Durchlassgitter 39 ab. Das Durchlassgitter 39 ist wiederum an der äußeren Gehäusewand 15 festgelegt. Die Kammer-Ventilsitz-Vorspannfeder 38 stellt eine Vorspanneinrichtung dar, die den Kammer-Ventilsitz 24 in Richtung auf eine nicht expandierte Stellung der Kammer 23 zu vorspannt.

Eine Verlagerungsbewegung des Hockdrtick-Ventilsitzes 17 längs der Längsachse 34 ist geführt durch Führungselemente 40, die mit dem Hochdruck-Ventilsitz 17 verbunden sind und sich stromabwärts von diesem durch das Durchlassgitter 21 und längs der Kammerhülse 26 sowie durch den Kammer-Ventilsitz 24 erstrecken. Die Führungselemente 40 tragen einen stromaufseitigen Kammer-Ventilsitz-Anschlag 41 und einen stromabseitigen Kammer-Ventilsitz-Anschlag 42.

Figur 3 zeigt den Beginn eines Füllvorgangs der Kammer 23, also einen Zeitpunkt kurz nach einem Überschreiten der Liquordnick-Obergrenze. Bei diesem Füllvorgang ist das aus dem Kammer-Ventilkörper 25, der dichtend auf dem Kammer-Ventilsitz 24 aufliegt, gebildete stromabseitige Kammerventil und damit auch das gesamte Ventil 7 geschlossen. Aufgrund des Liquordrucks in der Kammer 23 bewegt sich bei der durch die Kammerfüllung eingeleiteten Expansion der Kammer 23 das eine Art Kolben bzw. Dämpfungselement darstellende Kammerventil mit dem Kammer-Ventilkörper 25 und dem Kammer-Ventilsitz 24 in der Figur 3 nach rechts. Dabei bewegt sich auch die fest mit dem Kammer-Ventilsitz 24 verbundene Kammerhülse 26 in der Figur 3 nach rechts. Der Beginn dieses Verlagerungsvorganges ist in der Figur 3 dargestellt. Hierbei wird deutlich, dass die beiden Keilabschnitte 27, 28 als Entriegelungseinrichtung für die Verriegelungsvorrichtung 32 dienen. Durch die Verlagerung der Kammerhülse 26 in der Liquor-Strömungsrichtung 11 biegen die Hülsen-Keilabschnitte 27 die Feder-Keilabschnitte 28 nach außen, so dass in der Folge auch die Auswölbungen 30 nach außen gedrückt werden, wie in der Figur 3 dargestellt. Die Figur 3 zeigt die Stellung, in der die Verriegelungsvorrichtung 32 gerade noch eine Verlagerung des Hochdruck-Ventilsitzes 17 relativ zum Hochdruck-Ventilkörper 13 sperrt, da eine Verlagerung der freien Enden der Zapfen 31 in Liquor-Strömungsrichtung 11 gerade noch durch die sperrenden Auswölbungen 30 behindert ist.

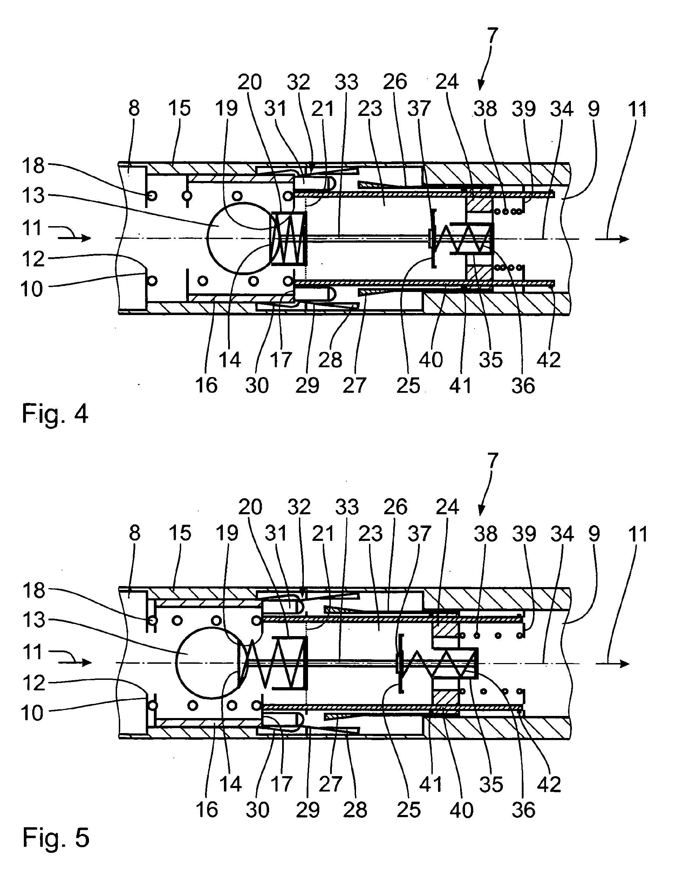

Figur 4 zeigt die Betriebssituation, bei der die Sperre durch die Verriegelungsvorrichtung 32 über die durch die Keilabschnitte 27, 28 gebildete Entriegelungseinrichtung überwunden ist. Aufgrund des Liquordrucks und der Vorspannkraft durch die Druckfeder 18, also durch die Hochdruck-Ventilsitz-Vorspannfeder, ist der Hochdruck-Ventilsitz 17 nun in der Liquor-Strömungsrichtung 11 verlagert.

Diese Verlagerung des Hochdruck-Ventilsitzes 17 in der Liquor-Strömungsrichtung 11 erfolgt, bis über die stromaufseitigen Kammer-Ventilsitz-Anschläge 41 an den Führungselementen 40, die den Kammer-Ventilsitz 24 mitnehmen, die Kammer-Ventilsitz-Vorspannfeder 38 vollständig komprimiert ist. Gegen den Druck der Druckfeder 18 wirkt zudem der Druck der Kammer-Ventilkörper-Vorspannfeder 36.

Aufgrund der Mitnahme des Kammer-Ventils 24 in der Liquor-Strömungsrichtung 11 ist das aus dem Kammer-Ventilkörper 25 und dem Kammer-Ventilsitz 24 gebildete stromabseitige Kammerventil des Ventils 7 geöffnet. Insgesamt ist nun eine Strömung von Flüssigkeit zwischen dem stromaufseitigen Abschnitt 8 und dem stromabseitigen Abschnitt 9 möglich. Wenn die Liquordruck-Obergrenze also so lange überschritten bleibt, dass durch ein Füllen der expandierbaren Kammer 23 sowohl der Hochdruck-Ventilsitz 17 vom Ventilkörper 13 abgehoben ist als auch das durch den Kammer-Ventilkörper 25 und den Kammer-Ventilsitz 24 gebildete Kammerventil geöffnet ist, öffnet das Ventil 7 insgesamt.

Figur 5 zeigt die Betriebssituation, bei der sich unter dem Einfluss der beiden Vorspannfedern 36 und 38 die Kammerhülse 26 wieder entgegen der Liquor-Strömungsrichtung 11 nach links verlagert hat. Gezeigt ist die Stellung kurz bevor über den Druck, den der Kammer-Ventilsitz 24 über die Führungselemente 40 auf den Hochdruck-Ventilsitz 17 ausübt, die Zapfen 31 wieder in die Auswölbungen 30 einrücken.

Über eine Vorgabe der Vorspannkraft der Ventilkörper-Vorspannfeder 14 ist eine Einstellung einerseits der Liquordruck-Untergrenze, bei der über ein Zusammenwirken des Ventilkörpers mit dem Niederdruck-Ventilsitz 10 das Ventil 7 schließen soll und andererseits eine Einstellung der Liquordruck-Obergrenze, bei der durch ein Zusammenwirken des Ventilkörpers 13 mit dem Hochdruck-Ventilsitz 17 das Ventil 7 schließen soll, möglich.

## Patentansprüche

1. Ventil (7) zum Einsatz in einer Leitung (3) zum Führen einer Flüssigkeit,
- mit einer Ausführung derart,
-- dass das Ventil (7) zwischen einer vorgegebenen Flüssigkeitsdruck-Untergrenze und einer vorgegebenen Flüssigkeitsdruck-Obergrenze geöffnet ist und beim Unterschreiten der Flüssigkeitsdruck-Untergrenze einerseits und beim Überschreiten der Flüssigkeitsdruck-Obergrenze andererseits zumindest zeitweilig und zumindest weitgehend geschlossen ist,
-- dass das Ventil (7) beim Überschreiten der Flüssigkeitsdruck-Obergrenze eine vorgegebene Zeitspanne geschlossen bleibt und dann bei anhaltendem Überschreiten der Flüssigkeitsdruck-Obergrenze wieder öffnet, **gekennzeichnet durch** einen Niederdruck-Ventilsitz (10), der mit einem Ventilkörper (13) derart zusammenwirkt, dass das Ventil (7) unterhalb der Flüssigkeitsdruck-Untergrenze geschlossen ist, und
- einen Hochdruck-Ventilsitz (17), der mit einem Ventilkörper (13) derart zusammenwirkt, dass das Ventil (7) oberhalb der Flüssigkeitsdruck-Obergrenze zumindest zeitweilig und zumindest weitgehend geschlossen ist.

2. Ventil nach Anspruch 1, **dadurch gekennzeichnet, dass** der Ventilkörper (13), der mit dem Niederdruck-Ventilsitz (10) zusammenwirkt, gleichzeitig der Ventilkörper ist, der mit dem Hochdruck-Ventilsitz (17) zusammenwirkt.

3. Ventil nach Anspruch 1 oder 2, **gekennzeichnet durch** eine Ventilkörper-Vorspannfeder (14), über die der Ventilkörper (13) in Richtung auf den Niederdruck-Ventilsitz (10) vorgespannt ist.

4. Ventil nach einem der Ansprüche 1 bis 3, **dadurch gekennzeichnet, dass** der Hochdruck-Ventilsitz (17) und der diesem zugeordnete Ventilkörper (13) derart gestaltet sind, dass in der mit dem Hochdruck-Ventilsitz (17) zusammenwirkenden Stellung des Ventilkörpers (13) ein im Vergleich zum geöffneten Ventil (7) geringer Flüssigkeitsstrom möglich ist,
wobei in Flüssigkeits-Strömungsrichtung (11) dem Hochdruck-Ventilsitz (17) nachgeordnet eine expandierbare und mit Flüssigkeit füllbare Kammer (23) angeordnet ist, die in der Flüssigkeits-Strömungsrichtung (11) über einen Kammer-Ventilsitz (24) der mit einem Kammer-Ventilkörper (25) zusammenwirkt, abschließt,
wobei
- bei durch den Hochdruck-Ventilkörper (13) verschlossenem Hochdruck-Ventilsitz (17) und nicht expandierter Kammer (23) der Kammer-Ventilkörper (25) den Kammer-Ventilsitz (24) verschließt,
- bei expandierter Kammer der Hochdruck-Ventilkörper (13) vom Hochdruck-Ventilsitz (17) einerseits und der Kammer-Ventilkörper (25) vom Kammer-Ventilsitz (24) andererseits abgehoben ist, so dass oberhalb der Flüssigkeitsdruck-Obergrenze ein Flüssigkeitsfluss in der Flüssigkeits-Strömungsrichtung (11) durch das Ventil (7) möglich ist.

5. Ventil nach Anspruch 4, **dadurch gekennzeichnet, dass** zumindest eines der Kammer-Ventilelemente (24, 25) über eine Vorspanneinrichtung (36, 38) in Richtung auf eine nicht expandierte Stellung der Kammer (23) zu vorgespannt ist.

6. Ventil nach Anspruch 4 oder 5, **dadurch gekennzeichnet, dass** der Hochdruck-Ventilkörper (13) mechanisch mit einem der Kammer-Ventilelemente (25) verbunden ist.

7. Ventil nach einem der Ansprüche 4 bis 6, **dadurch gekennzeichnet, dass** der Hochdruck-Ventilsitz (17) mechanisch mit einem der Kammer-Ventilelemente (24) verbunden ist.

8. Ventil nach Anspruch 6 oder 7, **dadurch gekennzeichnet, dass** eine Relativbewegung des mit einem der Hochdruck-Ventilelemente (13, 17) mechanisch verbundenen Kammer-Ventilelements (24, 25) relativ zum Hochdruck-Ventilelement (13, 17) möglich ist, wobei das mit dem Hochdruck-Ventilelement (13, 17) mechanisch verbundene Kammer-Ventilelement (24, 25) in Richtung auf einen dem Hochdruck-Ventilelement (13, 17) zugewandten Anschlag (37, 41) zu über eine Vorspanneinrichtung (36, 38) vorgespannt ist.

9. Ventil nach einem der Ansprüche 1 bis 8, **gekennzeichnet durch** eine Verringerungsvorrichtung (32), die
- in einer Sperrstellung eine Verlagerung des Hochdruck-Ventilsitzes (17) relativ zum Hochdruck-Ventilkörper (13) sperrt,
- in einer Freigabestellung eine Verlagerung des Hochdruck-Ventilsitzes (17) relativ zum Hochdruck-Ventilkörper (13) zur Freigabe einer Überströmverbindung freigibt.

10. Ventil nach Anspruch 9, **dadurch gekennzeichnet, dass** eines der Kammer-Ventilelemente (24, 25) mechanisch mit einer Entriegelungseinrichtung (27, 28, 29) für die Verriegelungsvorrichtung (32) verbunden ist.

11. Ventil nach Anspruch 9 oder 10, **gekennzeichnet durch** eine Hochdruck-Ventilsitz-Vorspannfeder (18), die den Hochdruck-Ventilsitz (17) in Richtung auf die Freigabestellung zu vorspannt.

12. Ventil nach Anspruch 10 oder 11, **dadurch gekennzeichnet, dass** die Entriegelungseinrichtung (27, 28, 29) eine Blattfeder (29) aufweist.

13. Ventil nach einem der Ansprüche 1 bis 12, **dadurch gekennzeichnet, dass** die Ventilkörper-Vorspannfeder (14) mit einstellbarer Vorspannkraft ausgeführt ist.

## Claims

1. Valve (7) for use in a conduit (3) for guiding a liquid,
- which is configured such that
-- the valve (7) is open between a predetermined lower limit of liquid pressure and a predetermined upper limit of liquid pressure and is closed at least temporarily and at least mostly if the lower limit of liquid pressure is fallen below on the one hand and the upper limit of liquid pressure is exceeded on the other hand,
-- the valve (7) remains closed if the upper limit of liquid pressure is exceeded for a predefined period and opens again if the upper limit of liquid pressure is exceeded for a sustained period,
**characterised by**
- a low pressure valve seat (10), which works together with a valve body (13) such that the valve (7) is closed below the lower limit of liquid pressure, and
- a high-pressure valve seat (17), which works together with a valve body (13) such that the valve (7) is closed at least temporarily and at least mostly above the upper limit of liquid pressure.

2. Valve according to claim 1, **characterised in that** the valve body (13) which cooperates with the low pressure valve seat (10) is at the same time the valve body which cooperates with the high-pressure valve seat (17).

3. Valve according to either claim 1 or 2, **characterised by** a valve body pretensioning spring (14), by means of which the valve body (13) is pretensioned in the direction of the low pressure valve seat (10).

4. Valve according to any one of claims 1 to 3, **characterised in that** the high-pressure valve seat (17) and the valve body (13) assigned thereto are configured in such a way that in the position of the valve body (13) cooperating with the high-pressure valve seat (17) a smaller liquid flow is possible than with an opened valve (7),
wherein in the liquid flow direction (11) downstream of the high-pressure valve seat (17) an expandable chamber (23) which can be filled with liquid is arranged, which in the liquid flow direction (11) closes via a chamber valve seat (24) which cooperates with a chamber valve body (25), wherein
- with a high-pressure valve seat (17) closed by the high-pressure valve body (13) and non-expanded chamber (23) the chamber valve body (25) closes the chamber valve seat (24),
- with an expanded chamber the high-pressure valve body (13) is lifted from the high-pressure valve seat (17) on the one hand and the chamber valve body (25) is lifted from the chamber valve seat (24) on the other hand, so that above the upper limit of liquid pressure a flow of liquid though the valve (7) is possible in liquid flow direction (11).

5. Valve according to claim 4, **characterised in that** at least one of the chamber valve elements (24, 25) is pretensioned by a pretensioning device (36, 38) in the direction of a non-expanded position of the chamber (23).

6. Valve according to either claim 4 or 5, **characterised in that** the high-pressure valve body (13) is connected mechanically to one of the chamber valve elements (25).

7. Valve according to any one of claims 4 to 6, **characterised in that** the high-pressure valve seat (17) is connected mechanically to one of the chamber valve elements (24).

8. Valve according to either claim 6 or 7, **characterised in that** a relative movement of the chamber valve element (24, 25) connected mechanically to one of the high-pressure valve elements (13, 17) is possible relative to the high-pressure valve element (13, 17), wherein the chamber valve element (24, 25) connected mechanically to the high-pressure valve element (13, 17) is pretensioned in the direction of a stop (37, 41) facing the high-pressure valve element (13, 17) by a pretensioning device (36, 38).

9. Valve according to any one of claims 1 to 8, **characterised by** a reduction device (32) which
- in a locked position blocks the displacement of the high-pressure valve seat (17) relative to the high-pressure valve body (13),
- in a release position allows the displacement of the high-pressure valve seat (17) relative to the high-pressure valve body (13) to open an overflow connection.

10. Valve according to claim 9, **characterised in that** one of the chamber valve elements (24, 25) is connected mechanically to an unlocking device (27, 28, 29) for the locking device (32).

11. Valve according to claim 9 or 10, **characterised by** a high-pressure valve seat pretensioning spring (18), which pretensions the high-pressure valve seat (17) in the direction of the release position.

12. Valve according to claim 10 or 11, **characterised in that** the unlocking device (27, 28, 29) comprises a leaf spring (29).

13. Valve according to any one of claims 1 to 12, **characterised in that** the valve body pretensioning spring (14) is configured to have an adjustable pretensioning force.

## Revendications

1. Soupape (7) à utiliser dans une conduite (3) pour diriger un liquide,
- comprenant une mise en oeuvre telle que
-- la soupape (7) est ouverte entre une limite inférieure de pression de liquide déterminée et une limite supérieure de pression de liquide déterminée et est fermée au moins temporairement et au moins largement lors du dépassement de la limite inférieure de la pression de liquide, d'une part, et lors du dépassement de la limite supérieure de la pression de liquide, d'autre part,
-- la soupape (7) reste fermée pendant un laps de temps donné lors du dépassement de la limite supérieure de la pression de liquide et ensuite s'ouvre de nouveau lors d'un dépassement prolongé de la limite supérieure de la pression du liquide,
**caractérisée par** un siège de soupape de basse pression (10) qui agit conjointement avec un corps de soupape (13) de telle sorte que la soupape (7) est fermée en dessous de la limite inférieure de la pression du liquide, et
- un siège de soupape de haute pression (17) qui agit conjointement avec un corps de soupape (13) de telle sorte que la soupape est au moins en deux pièces et est au moins largement fermée au dessus de la limite supérieure de pression de liquide.

2. Soupape selon la revendication 1 **caractérisée en ce que** le corps de soupape (13) qui agit conjointement avec le siège de soupape de basse pression (10) est simultanément le corps de soupape qui agit conjointement avec le siège de soupape de haute pression (17).

3. Soupape selon les revendications 1 ou 2 **caractérisée par** un ressort de précontrainte 14) du corps de soupape par lequel le corps de soupape (13) est précontraint en direction du siège de soupape de basse pression (10).

4. Soupape selon l'une des revendications de 1 à 3 **caractérisée en ce que** le siège de soupape de haute pression (17) et le corps de soupape (13) qui lui est attenant sont conçus de telle façon qu'un flux de liquide faible est possible dans la position du corps de soupape (13) agissant conjointement avec le siège de soupape de haute pression (17), en comparaison avec la position de soupape (7) ouverte,
une chambre (23) pouvant se dilater et pouvant être remplie de liquide, étant agencée après le siège de soupape de haute pression (17) dans la direction de l'écoulement du liquide (11), chambre se fermant dans la direction de l'écoulement du liquide (11) par l'intermédiaire d'un siège de soupape de chambre (24) qui agit conjointement avec un corps de soupape de la chambre (25),
où
- lorsque le siège de soupape de haute pression (17) est fermé par l'intermédiaire du corps de soupape de haute pression (13) et que la chambre (23) est non dilatée, le corps de soupape de chambre (25) ferme le siège de soupape de chambre (24),
- lorsque la chambre est en expansion, le corps de soupape de haute pression (13) du siège de soupape de haute pression (17), d'une part, et le corps de soupape de chambre (25) du siège de soupape de chambre (24), d'autre part, sont enlevés, de sorte qu'au dessus de la limite supérieure de la pression de liquide, un flux de liquide est possible par la soupape (7) dans la direction de l'écoulement du liquide (11).

5. Soupape selon la revendication 4 **caractérisée en ce qu'**au moins un élément de soupape de la chambre (24, 25) est précontraint par l'intermédiaire d'un dispositif de précontrainte (36, 38) en direction d'une position non dilatée de la chambre (23).

6. Soupape selon les revendications 4 ou 5 **caractérisée en ce que** le corps de soupape de haute pression (13) est relié mécaniquement avec l'un des éléments de soupape de la chambre (25).

7. Soupape selon l'une des revendications de 4 à 6 **caractérisée en ce que** le siège de soupape de haute pression (17) est relié mécaniquement avec l'un des éléments de soupape de la chambre (24).

8. Soupape selon les revendications 6 ou 7 **caractérisée en ce qu'**un mouvement relatif d'un des éléments de soupape de la chambre (24, 25) relié mécaniquement avec l'un des éléments de soupape de haute pression (17) est possible par rapport à l'élément de soupape de haute pression (13, 17), l'élément de soupape de la chambre (24, 25) relié mécaniquement avec l'élément de soupape de haute pression (13, 17) étant précontraint en direction d'une butée (37, 41) orientée vers l'élément de soupape de haute pression (13, 17) par l'intermédiaire d'un dispositif de précontrainte (36, 38).

9. Soupape selon l'une des revendications de 1 à 8 **caractérisée par** un dispositif de réduction (32) qui
- empêche un déplacement du siège de soupape de haute pression (17) par rapport au corps de soupape de haute pression (13) dans une position de verrouillage,
- libère le déplacement du siège de soupape de haute pression (17) par rapport au corps de soupape de haute pression (13) pour la libération d'une liaison de surintensité dans une position de libération.

10. Soupape selon la revendication 9 **caractérisée en ce qu'**un des éléments de soupape de la chambre (24, 25) est relié mécaniquement avec un dispositif de déverrouillage (27, 28, 29) pour le dispositif de verrouillage (32).

11. Soupape selon les revendications 9 ou 10 **caractérisée par** un ressort de précontrainte de siège de soupape de haute pression (18) qui précontraint le siège de soupape de haute pression (17) en direction du réglage en position de libération.

12. Soupape selon les revendications 10 ou 11 **caractérisée en ce que** le dispositif de déverrouillage (27, 28, 29) présente un ressort plat (29).

13. Soupape selon l'une des revendications de 1 à 12 **caractérisée en ce que** le ressort de précontrainte du corps de soupape (14) est conçu avec une force de précontrainte réglable.
